(19) Europäisches Patentamt
European Patent Office
Office européen des brevets

(11) Veröffentlichungsnummer : **0 150 006**
**B1**

# (12) EUROPÄISCHE PATENTSCHRIFT

(45) Veröffentlichungstag der Patentschrift : **10.09.86**

(51) Int. Cl.⁴ : **C 07 C121/75**// A01N53/00

(21) Anmeldenummer : **85100121.4**

(22) Anmeldetag : **07.01.85**

## (54) Verfahren zur Herstellung bestimmter Enantiomerenpaare von Permethrinsäure -alpha- cyano-3-phenoxy-4-fluorbenzyl-ester.

(30) Priorität : **18.01.84 DE 3401483**

(43) Veröffentlichungstag der Anmeldung : **31.07.85 Patentblatt 85/31**

(45) Bekanntmachung des Hinweises auf die Patenterteilung : **10.09.86 Patentblatt 86/37**

(84) Benannte Vertragsstaaten : **AT BE CH DE FR GB IT LI NL SE**

(56) Entgegenhaltungen :
**EP-A- 0 025 925**
**AT-B- 372 676**
**DE-A- 3 115 881**

(73) Patentinhaber : **BAYER AG**
**Konzernverwaltung RP Patentabteilung**
**D-5090 Leverkusen 1 Bayerwerk (DE)**

(72) Erfinder : **Fuchs, Rainer, Dr.**
**Roeberstrasse 8**
**D-5600 Wuppertal 1 (DE)**
Erfinder : **Naumann, Klaus, Dr.**
**Richard-Wagner-Strasse 9**
**D-5090 Leverkusen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 150 006 B1

**Beschreibung**

Die vorliegende Erfindung betrifft ein Verfahren zur Herstellung bestimmter Enantiomerenpaare von Permethrinsäure-α-cyano-3-phenoxy-4-fluor-benzylester ausgehend vom Gemisch aller sterischen und optischen Isomeren.

Es ist bekannt, daß Enantiomere von Verbindungen mit einem aciden Wasserstoffatom an einem asymmetrischen C-Atom durch Behandeln mit Basen epimerisiert werden können. Die bei der Reaktion mit Basen entstehenden Carbanionen gehen ständig rasch in ihre denkbaren enantiomeren Formen über. Sie passieren dabei kurzzeitig den ebenen Zustand (P. Sykes : Reaktionsaufklärung — Methoden und Kriterien der organischen Reaktionsmechanistik ; Verlag Chemie 1973, S. 133 und D. J. Cram : Fundamentals in Carbanion Chemistry S. 85-105, Academic Press New York 1965).

Dieser Fall wird z. B. auch bei der leichten basenkatalysierten Epimerisierung von optisch aktiver Mandelsäurenitril der Formel

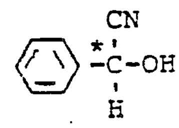

und des entsprechenden Methylethers der Formel

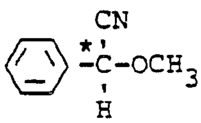

zu den razemischen Verbindungen beobachtet. (Smith : J. Chem. Soc. *1935*, S. 194 und Smith : Ber. *64* (1931) S. 427).

Je nach Löslichkeit der Gleichsgewichtspartner eines Epimerisierungsgleichgewichtes im labilen Diastereomeren kann das Gleichgewicht sehr stark oder vollständig auf eine Seite verschoben werden, wenn ein Teil auskristallisiert. Dieser Fall wird als « second order asymmetric transformation » bezeichnet. (K. Mislow, Introduktion to Stereochemistry, W. C. Benjamin Inc. New York, Amsterdam 1966, Seite 122 oben).

Dieser Effekt kann jedoch nur zur praktischen Anwendung gebracht werden wenn es gelingt, ein Lösungsmittel zu finden, in dem ein Stereoisomer und/oder sein Spiegelbild leichter und das andere Stereoisomer und/oder sein Spiegelbild schwerer löslich ist.

Eine solche Reaktion ist beispielsweise bereits mit dem optisch aktiven 2,2-Dimethyl-3R-(2,2-dichlorvinyl)-cyclopropan-1R-carbonsäure-α-cyano-(αRS)-3-phenoxybenzylester bekannt geworden (DE-OS 2 718 039). Als optimerisierende Basen werden Ammoniak und Amine eingesetzt. Als Lösungsmittel werden Acetonitril sowie niedere Alkanole eingesetzt. Dabei wird jedoch vom Ester eines bestimmten Enantiomeren der Carbonsäure (1R3R) ausgegangen.

Es fehlt jeder Hinweis darauf, daß dieses Verfahren auch eingesetzt werden kann um aus dem razemischen Gemisch aller 8 Stereoisomeren der obigen Verbindung eine Abtrennung von gewissen Stereoisomeren durch Epimerisierung der anderen zu erreichen.

Des weiteren ist aus DE-OS 2 903 057 bekannt, daß auch die 4 stereoisomeren α-Cyano-(αR,S)-3-phenonbenzylester einer razemischen Carbonsäure durch Behandlung mit Basen am α-Kohlenstoffatom neben der Cyanogruppe epimerisiert werden können und aus geeigneten Lösungsmitteln ein einzelnes Enantiomerenpaar auskristallisiert werden kann. Auch hier werden als geeignete Lösungsmittel niedere Alkohole insbesondere Methanol genannt. Als Base wird wäßriger Ammoniak eingesetzt.

In ähnlicher Weise verläuft nach EP-OS 22 382 ein Verfahren zur Umwandlung des Stereoisomerengemisches der 4 cis-Isomeren von Permethrinsäure-α-cyano-3-phenoxybenzylester in ein reines Enantiomerenpaar durch Auskristallisieren des schwerer löslichen Enantiomerenpaares in einem geeigneten Lösungsmittel, durch anschließendes Epimerisieren des in Lösung verbleibenden anderen Enantiomerenpaares mit einer Base und erneutes Auskristallisieren des schwerer löslichen Enantiomerenpaares. Kristallisieren und Epimerisieren werden dabei in getrennten Stufen vorgenommen. Als geeignetes Lösungsmittel werden dabei Kohlenwasserstoffe insbesondere Hexan offenbart. Als Basen werden Amine, insbesondere Triethylamin eingesetzt.

Aus DE-OS 3 115 881 ist ein weiteres Verfahren zur Umwandlung des Stereoisomerengemisches aller 4 cis-Isomeren von α-Cyano-3-phenoxybenzyl-3-(2,2-dichlorvinyl)-2,2-dimethylcyclopropancarbonsäureester in ein einzelnes Enantiomerenpaare bekannt geworden. Dabei wird als Lösungsmittel und gleichzeitig als Base ein organisches Amin verwendet. Als gut geeignet wird dabei Triethylamin und Diisopropylamin beschrieben. Als ungeeignet wird Tri-n-propylamin und n-Butylmethylamin genannt. Auch bei diesem Verfahren wird jedoch mit einem sterisch einheitlichen razemischen Säureteil (cis-Isomeren) gearbeitet. Auch hier fehlt jeder Hinweis darauf, ob eine Abtrennung einzelner Stereoisomeren

auch aus dem Gemisch aller 8 denkbaren Stereoisomeren möglich ist.

Es ist nicht möglich, Vorhersagen darüber zu machen, welche Lösungsmittel sich zur Trennung von Enantiomeren oder Diastereomeren der Enantiomerenpaaren eignen.

Es ist daher erforderlich für jede einzelne Verbindung ein geeignetes Trennsystem zu entwickeln. Erfahrungen aus prinzipiell ähnlich gelagerten Fällen lassen sich nur gelegentlich und dann in nicht vorhersehbarer Weise übertragen.

2,2-Dimethyl-3-dichlorvinyl-cyclopropan-carbonsäure-(Permethrinsäure)-α-cyano-3'-phenoxy-4'-fluorbenzylester besitzt die Strukturformel I

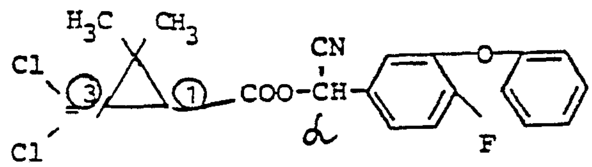

(I)

Die Verbindung besitzt drei asymmetrische Zentren①, ③ und α. Sie liegt damit in folgenden Enantiomerenpaaren vor :

| | |
|---|---|
| a : 1R-3R-αR + 1S-3S-αS<br>b : 1R-3R-αS + 1S-3S-αR | 1,3 cis |
| c : 1R-3S-αR + 1S-3R-αS<br>d : 1R-3S-αS + 1S-3R-αR | 1,3 trans |

Besonders wirksam gegen zahlreiche wirtschaftlich interessante Schädlinge sind die Enantiomerenpaare b und d.

Bei der technischen Herstellung der Verbindung der Formel I sind die Verhältnisse der Enantiomerenpaare a-d nur in einem gewissen engen Rahmen variabel. Bei einer typischen technisch hergestellten Verbindung der Formel I liegen die Enantiomerenpaare a-d z. B. in folgendem Verhältnis vor (bezogen auf 100 %)

a = 24,5 %
b = 17,5 %
c = 34,5 %
d = 23,5 %

Es sollte ein Verfahren gefunden werden, mit dem sich das Verhältnis der Enantiomerenpaare a-d im Gemisch aller Enantiomeren zugunsten der Enantiomerenpaare b und d verändert.

Es wurde gefunden, daß man aus dem Gemisch aller 8 Stereoisomeren der Verbindung Permethrinsäure-α-cyano-3-phenoxy-4-fluor-benzylester die Enantiomerenpaare

b) 1R-3R-αS + 1S-3S-αR und
d) 1R-3S-αS + 1S-3R-αR

dadurch abtrennen kann daß man dieses Gemisch aller Stereoisomeren in einem sekundären oder tertiären Alkylamin mit jeweils 2-6 C-Atomen je Alkylteil löst und aus der erhaltenen Lösung das Gemisch der Enantiomerenpaare b und d auskristallisiert.

Es war überraschend, daß Epimerisierung am C-Atom und Abtrennung der gewünschten Enantiomerenpaare mit demselben Lösungsmittel erreicht werden können. Es war außerdem überraschend, daß es für diesen Vorgang nicht erforderlich war, von einem im Säureteil sterisch einheitlichen Ester auszugehen, sondern daß eine Umwandlung des technisch anfallenden Gemisches aus allen 8 Stereoisomeren der cis und trans-Reihe in ein Gemisch, bestehend aus nur noch im wesentlichen 4 cis und trans Stereoisomeren gelang.

Das erfindungsgemäße Verfahren wird in sekundären oder tertiären Alkylaminen mit jeweils 2-6 C-Atomen je Alkylteil sowie ihren Gemischen durchgeführt. Bevorzugt seien Amine mit 3-4 C-Atomen je Alkylteil genannt. Insbesondere seien Di-iso-butylamin, Tri-iso-butylamin, Tri-n-butylamin sowie ihre Mischungen genannt.

Die Amine werden im wesentlichen wasserfrei eingesetzt. Das technische cis/trans-Ausgangsmaterial wird in der Aminbase bei 40-80 °C bevorzugt zwischen 50 und 70 °C gelöst. Man kühlt anschließend die Lösung auf —25 bis +30 °C ab. Die Kristallisation kann durch Zugabe einiger Kleinkristalle der Enantiomerenpaare b + d beschleunigt werden. Jedoch erfolgt die Kristallisation auch schon spontan. Die Isolierung der Enantiomerenpaare b + d erfolgt in üblicher Weise z. B. durch Filtration oder Zentrifugieren.

Die nachfolgenden Beispiele erläutern die Erfindung.

Für die Beispiele 1 und 2 wurde ein technisches Produkt folgender Zusammensetzung benutzt :

Ia = 24,5 %
Ib = 17,5 %
Ic = 34,5 %
Id = 23,5 %

Beispiel 1

10 g des technischen cis/trans Gemisches von allen 8 Isomeren α-Cyano-3-phenoxy-4-fluorbenzyl-3-(2,2-dichlorvinyl)-2,2-dimethyl-cyclopropancarbonsäureester wurden unter Erwärmen auf 50-70 °C in der in der Tabelle 1 aufgeführten Menge des organischen Amins gelöst. Die Lösung ließ man unter Rühren auf Raumtemperatur abkühlen und rührte dann bei 20 °C die in der Tabelle 1 angegebene Zeit. Der Kristallisation setzte meist spontan nach ca. 2-3 Stunden ein. Nur wo dies nicht der Fall war, wurden Impfkristalle der Enantiomerenpaare b und d (vgl. oben) zugesetzt.

Die entstandenen Kristalle wurden anschließend abgesaugt und mit wenig kaltem n-Hexan nachgewaschen. Die getrockneten Kristalle wurden gewogen und die Isomeren-Zusammensetzung durch HPLC (Hochdruckflüssigchromatographie) bestimmt. Die Ergebnisse sind in nachfolgender Tabelle zusammengefaßt.

(Siehe Tabelle Seite 5 f.)

Tabelle

| Amin | Menge Amin ml | Rührzeit in Std. | Ausbeute an Kristall. Produkt in g | Isomerenzusammensetzung in % Ia | Ib | Ic | Id |
|---|---|---|---|---|---|---|---|
| 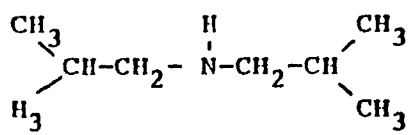 | 5 | 48 | 6,4 | 1,8 | 20,5 | 2,9 | 76,0 |
| | 10 | 48 | 5,2 | 1,5 | 13,3 | 1,8 | 83,3 |
| Di-iso-Butylamin | 15 | 48 | 4,7 | 0,9 | 12,8 | 1,1 | 85 |
| $(n-C_4H_9)_3N$ | 5 | 48 | 2,0 | - | 22,8 | 4,2 | 73 |
| | 10 | 48 | 3,0 | | | | |
| Tri-n-Butylamin | 40 | 48 | 4,4 | | | | |
| $N(C_2H_5)_3$ | 5 | 48 | 4,3 | 2,5 | 18,9 | 4,1 | 73,6 |
| | 10 | 48 | 4,1 | 2,1 | 18,2 | 2,8 | 76,9 |
| Triethylamin | 15 | 48 | 4,0 | 1,9 | 16,5 | 2,8 | 78,8 |

Beispiel 2

100 g eines technischen cis/trans-Gemisches aller 8 Stereoisomerer von α-Cyano-3-phenoxy-4-fluorbenzyl-3-(2,2-dichlorvinyl)-2,2-dimethyl-cyclopropancarbonester wurden unter Erwärmen auf 60 °C in 35 ml Di-iso-Butylamin gelöst. Unter Rühren ließ man langsam auf Raumtemperatur (20 °C) abkühlen. Hierbei setzte eine spontane Kristallisation ein. Anschließend wurde 48 Stunden bei 20 °C gerührt. Der entstandene kristalline Niederschlag wurde abgesaugt, mit 25 ml auf 0 °C abgekühltem n-Hexan nachgewaschen, trockengesaugt und dann an der Luft bis zur Gewichtskonstanz getrocknet. Man erhält 57 g eines farblosen kristallisierten Produkts mit einem Schmelzpunkt von 88-96 °C und folgender durch HPLC ermittelter Isomeren-Zusammensetzung (bezogen auf 100 %) :

a = 1,8 %, b = 19,7 %, c = 2,8 %, d = 76,9 %.

Das Filtrat wurde im Vakuum von Amin und n-Hexan befreit und das verbleibende Öl in 200 ml Toluol gelöst. Diese Lösung wurde mit 15 g Kieselgel 5 Minuten bei Raumtemperatur gerührt. Anschließend wurde abgesaugt und vom Filtrat im Vakuum das Toluol abdestilliert. Das verbleibende Öl (35 g) wurde bei 60 °C in einer Mischung von 14 g Di-iso-Butylamin und 56 g Tri-n-Butylamin gelöst. Nach Abkühlen auf 20 °C und 48 stündigem Rühren wurde wie oben aufgearbeitet.

Man erhält 11 g farblose Kristalle mit einem Schmelzpunkt von 69-72 °C und einer Isomeren-Zusammensetzung (HPLC-Methode) von :

a = 1,4 %, b = 77,2 %, c = 0,9 %, d = 19,4 %.

Das Filtrat aus der zweiten Stufe wurde nochmals wie das Filtrat aus der 1. Stufe behandelt. Man erhält so nochmals 8 g farblose Kristalle mit einem Schmelzpunkt von 71-72 °C, die überwiegend aus dem Isomeren b bestehen.

a = 1,2 %, b = 79,1 %, c = 0,8 %, d = 18,9 %.

Aus den 3 Stufen erhält man so insgesamt 76 g eines Gemisches der Isomeren b und d.

## Patentansprüche

1. Verfahren zur Herstellung der Enantiomerenpaare (1R-3R-αS + 1S-3S-αR) und (1R-3S-αS + 1S-3R-αR) aus dem Gemisch aller 8 Stereoisomeren der Verbindung αR/S-Cyano-3-phenoxy-4-fluorbenzyl-3R/S-(2,2-dichlorvinyl)-2,2-dimethyl-1R/S-cyclopropancarbonsäureester, dadurch gekennzeichnet, daß man dieses Gemisch in einem sekundären oder tertiären Alkylamin mit jeweils 2-6 C-Atomen je Alkylteil löst und aus der Lösung die Enantiomerenpaare (1R-3R-αS + 1S-3S-αR) und (1R-3S-αS + 1S-3R-αR) auskristallisiert und abtrennt.

2. Verfahren gemäß Anspruch 1, dadurch gekennzeichnet, daß man als Amin sekundäre oder tertiäre Amine mit 3-4 C-Atomen je Alkylgruppe einsetzt.

3. Verfahren gemäß Anspruch 1, dadurch gekennzeichnet, daß man als Amin Di-iso-butylamin und/oder Tri-n-butylamin einsetzt.

## Claims

1. Process for the preparation of the enantiomeric pairs (1R-3R-αS + 1S-3S-αR) and (1R-3S-αS + 1S-3R-αR) from a mixture of all 8 stereoisomers of the compound αR/S-cyano-3-phenoxy-4-fluorobenzyl 3R/S-(2,2-dichlorovinyl)-2,2-dimethyl-1R/S-cyclopropanecarboxylate, characterised in that this mixture is dissolved in a secondary or tertiary alkylamine having 2-6 C atoms per alkyl part, and the enantiomeric pairs (1R-3R-αS + 1S-3S-αR) and (1R-3S-αS + 1S-3R-αR) are crystallised out from the solution and are isolated.

2. Process according to Claim 1, characterised in that secondary or tertiary amines having 3-4 C atoms per alkyl group are employed as the amine.

3. Process according to Claim 1, characterised in that di-iso-butylamine and/or tri-n-butylamine are employed as the amine.

## Revendications

1. Procédé de fabrication des paires d'énantiomères (1R-3R-αS + 1S-3S-αR) et (1R-3S-αS + 1S-3R-αR) à partir du mélange de tous les 8 stéréoisomères du composé ester αR/S-cyano-3-phénoxy-4-fluorobenzylique d'acide 3R/S-(2,2-dichlorovinyl)-2,2-diméthyl-1R/S-cyclopropane-carboxylique, caractérisé en ce qu'on dissout ce mélange dans une alcoylamine secondaire ou tertiaire ayant chacune 2 à 6 atomes de carbone dans chaque portion alcoyle, et en ce qu'on cristallise et sépare à partir de la solution les paires d'énantiomères (1R-3R-αS + 1S-3S-αR) et (1R-3S-αS + 1S-3R-αR).

2. Procédé selon la revendication 1, caractérisé en ce qu'on utilise comme amine des amines secondaires ou tertiaires ayant 3 à 4 atomes de carbone dans chaque portion alcoyle.

3. Procédé selon la revendication 1, caractérisé en ce qu'on utilise en tant qu'amine de la di-isobutylamine et/ou de la tri-n-butylamine.